(19) Europäisches Patentamt
European Patent Office
Office européen des brevets

(11) Veröffentlichungsnummer: **0 573 883 A1**

(12) **EUROPÄISCHE PATENTANMELDUNG**

(21) Anmeldenummer: **93108786.0**

(51) Int. Cl.$^5$: **C07D 261/18**, A61K 31/42

(22) Anmeldetag: **01.06.93**

(30) Priorität: **12.06.92 DE 4219247**

(43) Veröffentlichungstag der Anmeldung: **15.12.93 Patentblatt 93/50**

(84) Benannte Vertragsstaaten: **AT BE CH DE DK ES FR GB IT LI NL**

(71) Anmelder: **BAYER AG**

**D-51368 Leverkusen(DE)**

(72) Erfinder: **Jeschke, Peter, Dr.**
**Heymannstrasse 38**
**W-5090 Leverkusen 1(DE)**
Erfinder: **Lindner, Werner, Dr.**
**Märchenstrasse 39**
**W-5000 Köln 80(DE)**
Erfinder: **Bonse, Gerhard, Dr.**
**Gerstenkamp 1**
**D 51061 Köln(DE)**
Erfinder: **Mencke, Norbert, Dr.**
**Grunder Mühle 2**
**W-5090 Leverkusen 3(DE)**
Erfinder: **Harder, Achim, Dr. Dr.**
**Piccolministrasse 398**
**W-5000 Köln 80(DE)**

(54) **Verwendung von 3-arylsubstituierten 5-Alkyl-isoxazol-4-carbonsäurederivaten zur Bekämpfung von Endoparasiten, neue 3-arylsubstituierte-5-Alkyl-isoxazol-4-carbonsäurederivate und Verfahren zu ihrer Herstellung.**

(57) Die vorliegende Erfindung betrifft die Verwendung von 3-arylsubstituierten 5-Alkyl-isoxazol-4-carbonsäurederivaten der allgemeinen Formel (I)

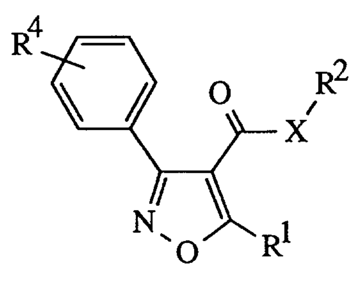

(I)

in welcher

$R^1$ für Alkyl oder Halogenalkyl steht,

$R^2$ für substituiertes $C_1$-$C_{20}$-Alkyl, $C_2$-$C_{18}$-Alkenyl, Cycloalkyl sowie für gegebenenfalls substituiertes Cycloalkylalkyl, Aralkyl, Heteroarylalkyl, Aryl, Heteroaryl steht, wobei als Substituenten genannten seien Halogen, Cyano, Nitro, Amino, Aminoacyl, Alkylamino, Alkyl, Halogenalkyl, Aralkyl, Hydroxy, Alkoxy, Halogenalkoxy, Aryloxy, Mercapto, Alkylthio, Halogenalkylthio, Alkylsulfinyl, Halogenalkylsulfinyl, Alkylsulfonyl, Halogenalkylsulfonyl, Aryl, Hetaryl, Arylthio oder Sulfonamidyl,

X für O, S, NH-NH- oder für den Rest -N-$R^3$ steht

$R^3$ für Wasserstoff, substituiertes $C_1$-$C_{20}$-Alkyl, $C_2$-$C_{18}$-Alkenyl, $C_1$-$C_6$-Halogenalkyl steht oder $R^2$ und $R^3$ gemeinsam mit dem angrenzenden N-Atom für einen 5-, 6 oder 7-gliedrigen Ring stehen, der gegebenenfalls auch durch O, S, N unterbrochen sein kann und gegebenenfalls durch $C_1$-$C_4$-Alkyl, Aralkyl oder Aryl substituiert ist,

EP 0 573 883 A1

$R^4$ für Wasserstoff, Halogen, Alkyl, Halogenalkyl steht,

zur Bekämpfung von Endoparasiten in der Medizin und Tiermedizin sowie neue Verbindungen der Formel (I) und deren Herstellung.

Die vorliegende Erfindung betrifft die Verwendung von 3-arylsubstituierten 5-Alkyl-isoxazol-4-carbonsäurederivaten zur Bekämpfung von Endoparasiten, neue 3-arylsubstituierte 5-Alkyl-isoxazol-4-carbonsäurederivate und Verfahren zur ihrer Herstellung.

Verschiedene 3-arylsubstituierte 5-Alkyl-isoxazol-4-carbonsäurederivate sind bereits als Verbindungen mit analgetischer und entzündungshemmender Aktivität bekannt geworden (vgl. DE-OS 3 247 454). Es ist jedoch nichts über ihre Verwendung gegen Endoparasiten bekannt.

Die vorliegende Erfindung betrifft:

1. Die Verwendung von 3-arylsubstituierten 5-Alkyl-isoxazol-4-carbonsäurederivaten der allgemeinen Formel (I)

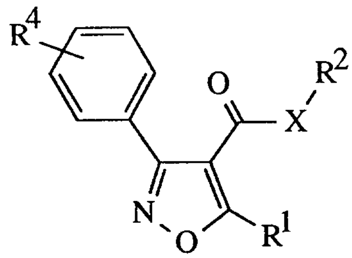
(I)

in welcher

$R^1$ für Alkyl oder Halogenalkyl steht,

$R^2$ für substituiertes $C_1$-$C_{20}$-Alkyl, $C_2$-$C_{18}$-Alkenyl, Cycloalkyl sowie für gegebenenfalls substituiertes Cycloalkylalkyl, Aralkyl, Heteroarylalkyl, Aryl, Heteroaryl steht, wobei als Substituenten genannt seien Halogen, Cyano, Nitro, Amino, Aminoacyl, Alkylamino, Alkyl, Halogenalkyl, Aralkyl, Hydroxy, Alkoxy, Halogenalkoxy, Aryloxy, Mercapto, Alkylthio, Halogenalkylthio, Alkylsulfinyl, Halogenalkylsulfinyl, Alkylsulfonyl, Halogenalkylsulfonyl, Aryl, Hetaryl, Arylthio oder Sulfonamidyl,

X für O,S, -NH-NH- oder für den Rest -N-$R^3$ steht

$R^3$ für Wasserstoff, substituiertes $C_1$-$C_{20}$-Alkyl, $C_2$-$C_{18}$-Alkenyl, $C_1$-$C_6$-Halogenalkyl steht oder $R^2$ und $R^3$ gemeinsam mit dem angrenzenden N-Atom für einen 5-, 6 oder 7-gliedrigen Ring stehen, der gegebenenfalls auch durch O, S, N unterbrochen sein kann und gegebenenfalls durch $C_1$-$C_4$-Alkyl, Aralkyl oder Aryl substituiert ist,

$R^4$ für Wasserstoff, Halogen, Alkyl, Halogenalkyl steht,

zur Bekämpfung von Endoparasiten in der Medizin und Tiermedizin.

Die Verbindungen der Formel (I) sind teilweise bekannt und lassen sich analog zu bekannten Verfahren herstellen.

2. Neue 3-arylsubstituierte 5-Alkyl-isoxazol-4-carbonsäurederivate der allgemeinen Formel (I)

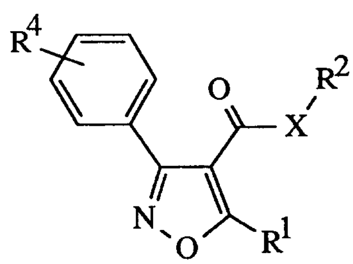
(I)

in welcher

$R^1$ für Alkyl oder Halogenalkyl steht,

$R^2$ für substituiertes $C_4$-$C_{20}$-Alkyl, $C_2$-$C_{18}$-Alkenyl, Cycloalkyl sowie für gegebenenfalls substituiertes Cycloalkylalkyl, Aralkyl, Heteroarylalkyl, Aryl, Heteroaryl steht, wobei als Substituenten genannten seien Cyano, Aminoacyl, Alkylamino, Aralkyl, Halogenalkoxy, Aryloxy, Mercapto, Alkylthio, Halogenalkylthio, Alkylsulfinyl, Halogenalkylsulfinyl, Alkylsulfonyl, Halogenalkylsulfonyl, Aryl, Hetaryl, Arylthio oder Sulfonamidyl,

X für O, S, -NH-NH- oder für den Rest -N-$R^3$ steht,

$R^3$ für Wasserstoff, substituiertes $C_1$-$C_{20}$-Alkyl, $C_2$-$C_{18}$-Alkenyl, $C_1$-$C_6$-Halogenalkyl steht oder $R^2$ und $R^3$ gemeinsam mit dem angrenzenden N-Atom für einen 5-, 6 oder 7-gliedrigen Ring stehen, der gegebenenfalls auch durch O, S, N unterbrochen sein kann und gegebenenfalls durch $C_1$-$C_4$-Alkyl, Aralkyl oder Aryl substituiert ist,

$R^4$ für Wasserstoff, Halogen, Alkyl, Halogenalkyl steht.

3. Verfahren zur Herstellung der neuen 3-arylsubstituierten 5-Alkyl-isoxazol-4-carbonsäurederivate der Formel (I)

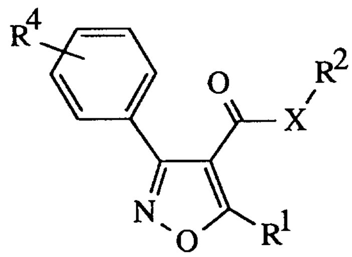 (I)

in welcher

X, $R^1$, $R^2$, $R^3$ und $R^4$ die unter Punkt 2 angegebene Bedeutung besitzen,

dadurch gekennzeichnet, daß man

a) Verbindungen der Formel (II)

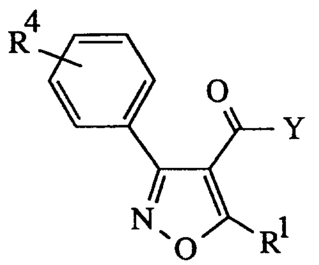 (II)

in welcher

$R^1$, $R^4$ die oben angegebene Bedeutung besitzen und

Y für eine geeignete Abgangsgruppe steht,

mit Verbindungen der Formel (III)

$R^2$-X-H (III)

in welcher

X, $R^2$ die oben angegebene Bedeutung besitzen,

umsetzt.

Die Verbindungen der Formel (I) eignen sich hervorragend zur Bekämpfung von Endoparasiten, besonders auf dem Gebiet der Veterinärmedizin.

Die erfindungsgemäßen 3-arylsubstituierten 5-Alkyl-isoxazol-4-carbonsäurederivate sind durch die Formel (I) allgemein definiert.

Bevorzugt sind Verbindungen der Formel (I), in welcher

$R^1$ für $C_1$-$C_4$-Alkyl oder $C_1$-$C_4$-Halogenalkyl steht,

$R^2$ für substituiertes $C_1$-$C_{20}$-Alkyl, $C_2$-$C_{18}$-Alkenyl, $C_3$-$C_7$-Cycloalkyl sowie für gegebenenfalls substituiertes $C_3$-$C_7$-Cycloalkyl-$C_1$-$C_2$-alkyl, Phenyl-$C_1$-$C_2$-alkyl,Heteroaryl-$C_1$-$C_2$-alkyl, Phenyl, Heteroaryl steht, wobei als Substituenten genannt seien eine oder mehrere gleiche oder verschiedene Reste der Gruppe $C_1$-$C_4$-Alkyl, insbesondere Methyl, Ethyl oder Isopropyl, $C_1$-$C_4$-Halogenalkoxy, insbesondere Trifluormethyl, $C_1$-$C_4$-Alkoxy, insbesondere Methoxy, Ethoxy, Methylendioxy oder Ethylendioxy, die gegebenenfalls durch Fluor oder Chlor substituiert sind, $C_1$-$C_4$-Halogenalkoxy, insbesondere Trifluormethoxy, Fluorchlorethoxy, $C_1$-$C_4$-Alkylthio, insbesondere Methylthio, $C_1$-$C_4$-

Halogenalkylthio, insbesondere Trifluormethylthio, Fluorchlormethylthio, $C_1$-$C_4$-Alkylsulfonyl, insbesondere Methylsulfonyl, $C_1$-$C_4$-Halogenalkylsulfonyl, insbesondere Trifluormethylsulfonyl, Halogen, insbesondere Fluor, Chlor oder Brom, Cyano, Nitro, Hydroxy, Mercapto, Amino, $C_1$-$C_4$-Alkylamino, $C_1$-$C_4$-Dialkylamino, insbesondere Dimethylamino, Acylamino, insbesondere Acetylamino, Phenyl, Phenylthio, Phenoxy, Heteroaryl, die gegebenenfalls durch einen oder mehrere gleiche oder verschiedene der oben genannten Reste substituiert sind,

X für O, S, -NH-NH- oder für den Rest -N-$R^3$ steht,

$R^3$ für Wasserstoff, substituiertes $C_1$-$C_{20}$-Alkyl, $C_2$-$C_{18}$-Alkenyl, $C_1$-$C_6$-Halogenalkyl steht oder $R^3$ und $R^2$ gemeinsam mit dem angrenzenden N-Atom für einen 5-, 6- oder 7-gliedrigen Ring stehen, der gegebenenfalls auch durch O, S, N unterbrochen sein kann und gegebenenfalls durch $C_1$-$C_4$-Alkyl, Phenyl-$C_1$-$C_2$-alkyl und Phenyl substituiert ist,

$R^4$ für Wasserstoff, Halogen, insbesondere Fluor, Chlor oder Brom, $C_1$-$C_4$-Alkyl, insbesondere Methyl, Ethyl oder Isopropyl, $C_1$-$C_4$-Alkoxy, insbesondere Methoxy oder Ethoxy, $C_1$-$C_4$-Halogenalkyl, insbesondere Trifluormethyl, $C_1$-$C_4$-Halogenalkoxy, insbesondere Trifluormethoxy, $C_1$-$C_4$-Halogenalkylthio steht.

Als Heteroaryl sei genannt Pyridyl, Chinolyl, Thiophenyl.

Besondere bevorzugt sind Verbindungen der Formel (I)

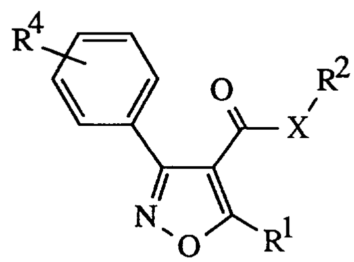

(I)

in welcher

$R^1$ für $C_1$-$C_4$-Alkyl, insbesondere Methyl, Ethyl, Propyl, Isopropyl, Butyl, Isobutyl, sec-Butyl, tert.-Butyl oder $C_1$-$C_4$-Halogenalkyl, insbesondere Trifluormethyl steht,

$R^2$ für substituiertes $C_1$-$C_{20}$-Alkyl, $C_2$-$C_{18}$-Alkenyl, insbesondere Methyl, Ethyl, Propyl, Isopropyl, Butyl, Isobutyl, sec.-Butyl, tert.-Butyl, Hexyl, Dodecyl, Tetradecyl, Hexadecyl, Oktadecyl oder Nonadecyl, $C_3$-$C_7$-Cycloalkyl, insbesondere Cyclopentyl, Cyclohexyl oder Cycloheptyl, Phenyl, Heteroaryl, insbesondere Pyridyl oder Chinolyl steht, die gegebenenfalls substituiert sind durch Wasserstoff, Halogen, insbesondere Fluor, Chlor oder Brom, $C_1$-$C_4$-Alkyl, insbesondere Methyl oder Ethyl, $C_1$-$C_4$-Halogenalkyl, insbesondere Trifluormethyl, $C_1$-$C_4$-Halogenalkoxy, insbesondere Trifluormethoxy oder Fluorchlorethoxy, $C_1$-$C_4$-Halogenalkylthio, insbesondere Trifluormethylthio, $C_1$-$C_4$-Halogenalkylsulfonyl, insbesondere Trifluormethylsulfonyl steht,

X für O, S, -NH-NH- oder für den Rest -N-$R^3$ steht,

$R^3$ für Wasserstoff, substituiertes $C_1$-$C_{20}$-Alkyl, insbesondere Methyl, Ethyl, Propyl, Butyl, Pentyl, Hexyl, Heptyl, Oktyl, Nonyl, Decyl, Dodecyl, Hexadecyl, Tetradecyl, Oktadecyl, $C_2$-$C_{18}$-Alkenyl, insbesondere Oktadecyl, $C_1$-$C_6$-Halogenalkyl, insbesondere Trifluormethyl, Pentafluorethyl steht oder $R^3$ und $R^2$ gemeinsam mit dem angrenzenden N-Atom für einen 5-, 6- oder 7-gliedrigen Ring stehen der gegebenenfalls auch durch O, S, N unterbrochen sein kann und gegebenenfalls durch $C_1$-$C_4$-Alkyl, insbesondere Methyl, Phenyl-$C_1$-$C_4$-alkyl, insbesondere Benzyl, Aryl, insbesondere Phenyl, die substituiert sind durch einen oder mehrere gleiche oder verschiedene der oben genannten Reste, substituiert ist,

$R^4$ für Wasserstoff, Halogen, insbesondere Fluor, Chlor oder Brom, $C_1$-$C_4$-Alkyl, insbesondere Methyl, $C_1$-$C_4$-Halogenalkyl, insbesondere Trifluormethyl steht.

Ganz besonders bevorzugt sind Verbindungen der Formel (I),

in welcher

$R^1$ für $C_1$-$C_4$-Alkyl, insbesondere Methyl, Ethyl, Propyl, oder Isopropyl steht.

$R^2$ für $C_1$-$C_{20}$-Alkyl, insbesondere Dodecyl, Tetradecyl, Hexadecyl, Oktadecyl, oder Nonadecyl, Phenyl, Heteroaryl, insbesondere Pyridyl steht, die gegebenenfalls substituiert sind durch Wasserstoff, Halogen, insbesondere Fluor oder Chlor, $C_1$-$C_4$-Alkyl, insbesondere Methyl, $C_1$-$C_4$-Halogenalkyl, insbesondere Trifluormethyl, $C_1$-$C_4$-Halogenalkoxy, insbesondere Trifluormethoxy,

$C_1$-$C_4$ Halogenalkylthio, insbesondere Trifluormethylthio,

X für O, S, -NH-NH- oder für den Rest -N-$R^3$ steht,

$R^3$ für Wasserstoff oder $C_1$-$C_{20}$-Alkyl, insbesondere Methyl, Dodecyl, Hexadecyl, Tetradecyl, Hexadecyl, Oktadecyl steht,

$R^4$ für Wasserstoff, Halogen, insbesondere Fluor oder Chlor steht.

Im einzelnen seien folgende Verbindungen der Formel (I) genannt:

3-Phenyl-5-methyl-isoxazol-4-carbonsäure-(4-chlor-phenyl)-ester,
N-(4-Methyl-phenyl)-3-phenyl-5-methyl-isoxazol-4-carbonsäureamid,
N-(2,3-Dimethyl-phenyl)-3-phenyl-5-methyl-isoxazol-4-carbonsäureamid,
N-(4-Chlor-phenyl)-3-pheny-5-methyl-isoxazol-4-carbonsäureamid,
N-(4-Brom-phenyl)-3-phenyl-5-methyl-isoxazol-4-carbonsäureamid,
N-(4-Trifluormethyl-phenyl)-3-phenyl-5-methyl-isoxazol-4-carbonsäureamid,
N-(2,5-Difluor-phenyl)-3-phenyl-5-methyl-isoxazol-4-carbonsäureamid,
3-(2-Chlor-phenyl)-5-methyl-isoxazol-4-carbonsäure-butylester,
3-(2-Chlor-phenyl)-5-methyl-isoxazol-4-carbonsäure-isopropylester,
3-(2-Chlor-phenyl)-5-methyl-isoxazol-4-carbonsäure-(4-methoxy-phenyl)-ester,
3-(2-Chlor-phenyl)-5-methyl-isoxazol-4-carbonsäure-(4-chlor-phenyl)-ester,
3-(2-Chlor-phenyl)-5-methyl-isoxazol-4-carbonsäure-(2-methyl-phenyl)-ester,
3-(2-Chlor-phenyl)-5-methyl-isoxazol-4-carbonsäure-(3-trifluormethyl-phenyl)-ester,
N-Butyl-3-(2-chlor-phenyl)-5-methyl-isoxazol-4-carbonsäureamid,
N-Cyclopentyl-3-(2-chlor-phenyl)-5-methyl-isoxazol-4-carbonsäureamid,
N-Oktadecyl-3-(2-chlor-phenyl)-5-propyl-isoxazol-4-carbonsäureamid,
N-(2-Methyl-3-chlor-phenyl)-3-(2-chlor-phenyl)-5-methyl-isoxazol-4-carbonsäureamid,
N-Oktadecyl-3-(2-chlor-phenyl)-5-isopropyl-isoxazol-4-carbonsäureamid,
N-(2-Methyl-phenyl)-3-(4-chlor-phenyl)-5-methyl-isoxazol-4-carbonsäureamid,
N-(4-Methyl-phenyl)-3-(4-chlor-phenyl)-5-methyl-isoxazol-4-carbonsäureamid,
N-(3-Chlor-phenyl)-3-(4-chlor-phenyl)-5-methyl-isoxazol-4-carbonsäureamid,
3-(2,6-Dichlor-phenyl)-5-methyl-isoxazol-4-carbonsäure-isopropylester,
3-(2,6-Dichlor-phenyl)-5-methyl-isoxazol-4-carbonsäure-butylester,
N-Acetyl-3-(2,6-dichlor-phenyl)-5-methyl-isoxazol-4-carbonsäureamid,
3-(2,6-Dichlor-phenyl)-5-methyl-isoxazol-4-carbonsäure-(3-trifluormethyl-phenyl)-ester,
N-Cyclopentyl-3-(2,6-dichlor-phenyl)-5-methyl-isoxazol-4-carbonsäureamid,
N-Octadecyl-3-(2,6-dichlor-phenyl)-5-propyl-isoxazol-4-carbonsäureamid,
N-Octadecyl-3-(2,6-dichlor-phenyl)-5-isopropyl-isoxazol-4-carbonsäureamid,
N-(4-Methyl-phenyl)-3-(2,6-dichlor-phenyl)-5-ethyl-isoxazol-4-carbonsäureamid,
N-(2,3-Dichlor-phenyl)-3-(2,6-dichlor-phenyl)-5-ethyl-isoxazol-4-carbonsäureamid,
N-(4-Fluor-phenyl)-3-(2,6-dichlor-phenyl)-5-methyl-isoxazol-4-carbonsäurehydrazid,
N-(2,4-Difluor-phenyl)-3-(2,6-dichlor-phenyl)-5-methyl-isoxazol-4-carbonsäurehydrazid,
N-(2,5-Difluor-phenyl)-3-(2,6-dichlor-phenyl)-5-methyl-isoxazol-4-carbonsäurehydrazid,
3-(2-Chlor-6-fluor-phenyl)-5-methyl-isoxazol-4-carbonsäure-isopropylester,
3-(2-Chlor-6-fluor-phenyl)-5-methyl-isoxazol-4-carbonsäure-hexadecylester,
3-(2-Chlor-6-fluor-phenyl)-5-propyl-isoxazol-4-carbonsäure-hexadecylester,
3-(2-Chlor-6-fluor-phenyl)-5-methyl-isoxazol-4-carbonsäure-(4-methoxy-phenyl)-ester,
3-(2-Chlor-6-fluor-phenyl)-5-methyl-isoxazol-4-carbonsäure-(3-trifluormethyl-phenyl)-ester,
N-Oktadecyl-3-(2-chlor-6-fluor-phenyl)-5-propyl-isoxazol-4-carbonsäureamid,
N-Oktadecyl-3-(2-chlor-6-fluor-phenyl)-5-isopropyl-isoxazol-4-carbonsäureamid,
N-(2-Trifluormethoxy-phenyl)-3-(2-chlor-6-fluor-phenyl)-5-methyl-isoxazol-4-carbonsäureamid,
N-(3-Chlor-2-methyl-phenyl)-3-(2-chlor-6-fluor-phenyl)-5-methyl-isoxazol-4-carbonsäureamid,
N-(4-Trifluormethylthio-phenyl)-3-(2-chlor-6-fluor-phenyl)-5-methyl-isoxazol-4-carbonsäureamid,
N-(4-Chlor-phenyl)-3-(2-chlor-6-fluor-phenyl)-5-methyl-isoxazol-4-carbonsäurehydrazid.

Von den neuen Verbindungen der Formel (I) sind diejenigen bevorzugt und besonders bevorzugt in denen die Substituenten die oben angegebenen bevorzugten Definitionen besitzen.

Die Verbindungen der Formel (I) sind teilweise bekannt, sie lassen sich nach dem oben unter Punkt 3 angegebenen Verfahren a) herstellen (vgl. z.B.: R.A. Barnes, Heterocyclic Compounds (Edited by R.C. Elderfield), Vol. 5, 1957, John Wiley & Sons. Inc. New York, S. 452; "Schotten-Baumann-Reaktion" in Houben-Weyl, Methoden der organischen Chemie, Bd. VIII, 4. Aufl., 1952, G Thieme Verlag Stuttgart-New York. S. 655; K. Dietliker et al. Helv. Chim. Acta 59 (1976) 6, S. 2074; J.C. Hanson et al. J. Chem. Soc. (1965) S. 5976; F.P. Doyle et al. J. Chem. Soc. (1963) S. 5845; F.P. Doyle et al. J. Chem. Soc. (1963) S.

5838; C.S. Nion et al. Heterocycles 24 (1986) 2, S. 401: EP 0 418 667; EP 84 292; PL 122 813, ref. C.A. 104 (15), 129708 g; DE-OS 3 247 454).

Setzt man bei Verfahren 3a zur Herstellung der neuen 3-arylsubstituierten 5-Alkyl-isoxazol-4-carbonsäurederivate als Verbindungen der Formel (II) 3-(2,6-Dichlor-phenyl)-5-ethyl-isoxazol-4-carbonsäurechlorid und als Verbindungen der Formel (III) n-Oktadecylamin ein, so läßt sich das Verfahren durch folgendes Reaktionsschema wiedergeben:

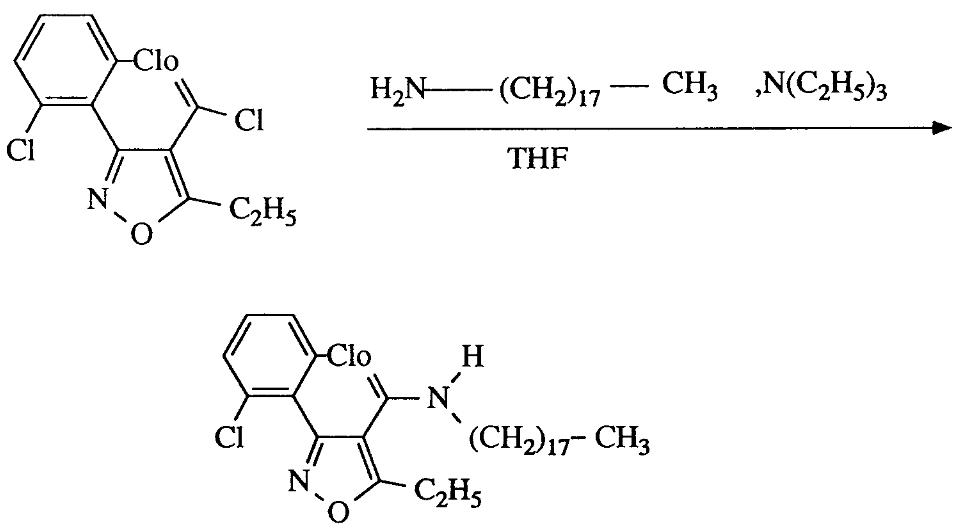


Die zur Durchführung des erfindungsgemäßen Verfahrens 3a als Ausgangsstoffe benötigten 3-Aryl-5-alkyl-isoxazol-4-carbonsäurehalogenide sind durch die Formel (II) allgemein definiert. In dieser Formel stehen $R^2$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden. In der Formel (II) hat Y die Bedeutung einer geeigneten Abgangsgruppe wie beispielsweise Halogen oder Alkoxy.

Die als Ausgangsmaterialien verwendeten Verbindungen der Formel (II) sind teilweise bekannt und können nach bereits in der Synthese von 3-Aryl-5-alkyl-4-isoxazolyl-penicillinen angewandten Methoden (vgl. z.B. J.C. Hanson et al. J. Chem. Soc. (1965). S. 5976; F.P. Doyle et al. J. Chem. Soc. (1963), S. 5845; F.P. Doyle et al. J. Chem. Soc. (1963), S. 5838; US-Pat. 3 534 020; US-Pat. 3 466 296) erhalten werden.

Im nachfolgenden Reaktionsschema wird die Darstellung von Verbindungen der Formel (II) wiedergegeben, wobei die Reste $R^2$ und $R^4$ die gleiche Bedeutung wie oben besitzen.

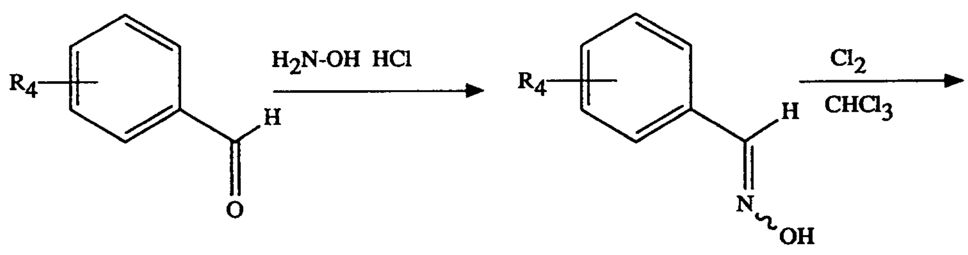


(IV) (V)

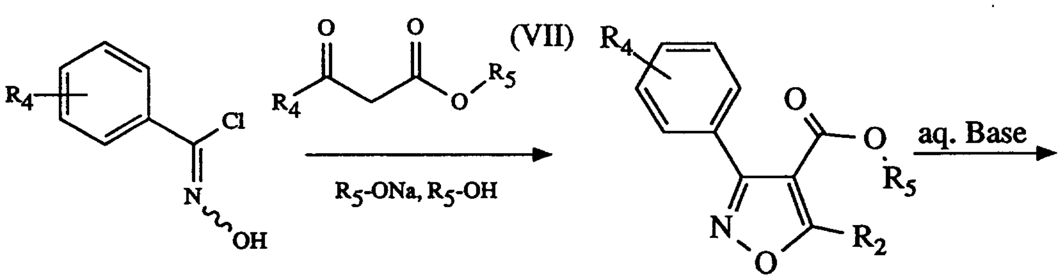


(VI) (VIII)

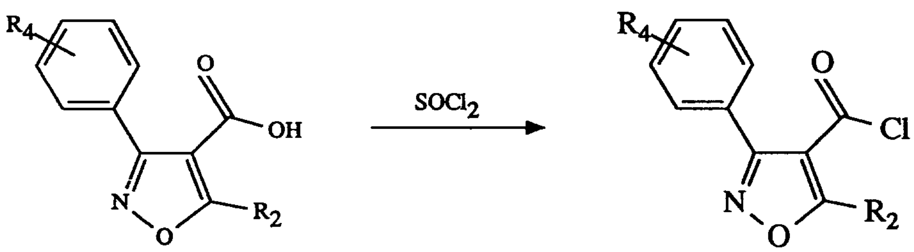


(IX) (II)

Die benötigten Aldoxime der allgemeinen Formel (V) sind entweder bekannt oder können nach an sich bekannten Verfahren (z.B. Houben-Weyl, Methoden der organischen Chemie, Bd. X/4, 4. Aufl., 1968, G. Thieme Verlag, Stuttgart New York, S. 55; Bd. E 14 b, 4. Aufl. 1990, G. Thieme Verlag, Stuttgart-New York, S. 287; J.P. Freemann Chem. Rev. 73 (1973), S. 283) durch Umsetzung der entsprechenden Aldehyde (IV) mit Hydroxylamin, hergestellt werden. Ihre Chlorierung zu Verbindungen der allgemeinen Formel (VI) wurde durchgeführt, indem man Verbindungen der allgemeinen Formel (V) in Chloroform löste und eine Lösung von Chlor in Chloroform zugab (vgl. Houben-Weyl, Methoden der organischen Chemie, Bd. VIII, 4. Aufl., 1952, G. Thieme Verlag, Stuttgart-New York, S. 691; Bd. X/3, 4. Aufl. 1965, G. Thieme Verlag, Suttgart-New York, S. 847).

Die Aldoxime (V) und die Hydroximsäurechloride (VI) können selbstverständlich sowohl in Form ihrer E- oder Z-Isomeren als auch Gemische dieser Stereoisomeren verwendet werden. Die Reaktion der so erhaltenen Verbindungen mit Natriumacylessigsäurealkylester (VII) lieferte die Alkylester der 3-Aryl-5-alkyl-isoxazol-4-carbonsäurender allgemeinen Formel (VIII). Als Isoxazol-4-carbonsäure-alkylester (VIII) eignen sich insbesondere Niedrigalkylester ($R^5$ = $C_1$-$C_4$-Alkyl), wobei Methylester und Ethylester besonders bevorzugt sind. Die Verseifung zur Isoxazol-4-carbonsäure (IX) wird bevorzugt in Gegenwart einer starken Base, z.B. NaOH, KOH oder $Ca(OH)_2$ durchgeführt. Im allgemeinen wird dabei etwa 1 Äquivalent der starken Base in wäßriger Lösung eingesetzt. Nach erfolgter Umsetzung wird mit einer starken Mineralsäure, z.B. Salzsäure oder Schwefelsäure, angesäuert. Zur Überführung der Carbonsäure (IX) in das Carbonsäurehalogenid (II, Y = Cl) bringt man die Carbonsäure (IX) in üblicher Art und Weise mit einem organischen

Säurehalogenid wie Thionylchlorid, Phosphortri- oder Phosphorpentahalogeniden, zur Reaktion, wobei die Chloride bevorzugt sind (vgl. Houben-Weyl, Methoden der organischen Chemie, Bad. VIII, 4. Aufl. 1952, G. Thieme Verlag Stuttgart-Yew York, S. 359).

Im einzelnen seien folgende Verbindungen der Formel (II) genannt, in welcher die Reste $R^2$, $R^4$ und Y die folgende Bedeutung haben:

3-Phenyl-5-methyl-isoxazol-4-carbonsäurechlorid,
3-Phenyl-5-ethyl-isoxazol-4-carbonsäurechlorid,
3-(2-Chlor-phenyl)-5-methyl-isoxazol-4-carbonsäurechlorid,
3-(2-Chlor-phenyl)-5-ethyl-isoxazol-4-carbonsäurechlorid,
3-(2-Chlor-phenyl)-5-propyl-isoxazol-4-carbonsäurechlorid,
3-(4-Chlor-phenyl)-5-methyl-isoxazol-4-carbonsäurechlorid,
3-(4-Chlor-phenyl)-5-ethyl-isoxazol-4-carbonsäurechlorid,
3-(4-Chlor-phenyl)-5-propyl-isoxazol-4-carbonsäurechlorid,
3-(4-Chlor-phenyl)-5-isopropyl-isoxazol-4-carbonsäurechlorid,
3-(2,6-Dichlor-phenyl)-5-methyl-isoxazol-4-carbonsäurechlorid,
3-(2,6-Dichlor-phenyl)-5-ethyl-isoxazol-4-carbonsäurechlorid,
3-(2,6-Dichlor-phenyl)-5-propyl-isoxazol-4-carbonsäurechlorid,
3-(2,6-Dichlor-phenyl)-5-isopropyl-isoxazol-4-carbonsäurechlorid,
3-(2-Chlor-6-fluor-phenyl)-5-methyl-isoxazol-4-carbonsäurechlorid,
3-(2-Chlor-6-fluor-phenyl)-5-ethyl-isoxazol-4-carbonsäurechlorid,
3-(2-Chlor-6-fluor-phenyl)-5-propyl-isoxazol-4-carbonsäurechlorid,
3-(2-Chlor-6-fluor-phenyl)-5-isopropyl-isoxazol-4-carbonsäurechlorid.

Die außerdem für die Durchfuhrung des erfindungsgemäßen Verfahrens 3a an Ausgangstoffen zu verwendenden Amino-, Hydrazino-, Mercapto- oder Hydroxyverbindungen der Formel (III) sind allgemein definiert. In dieser Formel (III) haben $R^1$, $R^3$ und X die Bedeutung, die bereits im Zusammenhang mit der Beschreibung der erfindungsgemäßen Stoffe der Formel (I) als bevorzugt für diese Substituenten genannt wurden. Die Amino-, Hydrazino-, Mercapto- oder Hydroxyverbindungen der Formel (III) sind in den meisten Fällen kommerziell erhältlich.

Die Reaktion der Verbindungen der Formel (II) und (III) wird vorzugsweise unter Verwendung von Verdünnungsmitteln und in Gegenwart eines basischen Reaktionshilfsmittels durchgeführt.

Als Verdünnungsmittel zur Durchführung des erfindungsgemäßen Verfahrens 3a kommen alle inerten organischen Lösungsmittel in Frage.

Als Beispiele sind zu nennen: Halogenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Di-n-butylether, Di-isobutylether, Diisoamlyether, Diisopropylether, Anisol, Phenetol, Cyclohexylmethylether, Diethylether, Ethylenglykoldimethylether, Tetrahydrofuran, Dioxan, Dichlordiethylether, Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol. Benzinfraktionen innerhalb eines Siedepunktintervalles von 70 °C bis 190 °C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methyl-pyrrolidon; Ketone wie Aceton, Methylethylketon. Auch Gemische der genannten Lösungs- und Verdünnungsmittel kommen in Betracht.

Bevorzugt sind Ether wie Tetrahydrofuran und Dioxan.

Als basische Reaktionshilfsmittel können alle geeigneten Säurebindmittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidon, N-Methyl-piperidin, N-Methyl-imidazol, N-Methyl-pyrrol, N-Methyl-morpholin, N-Methyl-hexamethylenimin, Pyridin, Chinolin, α-Picolin, β-Picolin, Isochinolin, Pyrimidin, Acridin, N,N,N',N'-Tetra-methylendiamin, N,N,N',N'-Tetra-ethylendiamin, Chinoxalin, N-Propyl-diisopropylamin, N,N'-Dimethyl-cyclohexylamin, 2,6-Lutidin, 2,4-Lutidin, Triethylendiamin, Diazabicyclooctan (DABCO), Diazabicyclononen (DBN) oder Diazabicycloundecen DBU).

Vorzugsweise verwendet man Hydroxide des Natriums und Kaliums oder tertiäre Amine, wie beispielsweise Triethylamin, Tribenzylamin oder Trihexylamin.

Das Verfahren 3a wird durchgeführt, indem Verbindungen der Formel (II) und Verbindungen der Formel (III) in Gegenwart eines Überschusses eines der angegebenen basischen Reaktionshilfsmittel in einem der angegebenen Verdünnungsmittel zusammengegeben und gerührt bzw. gegebenenfalls erhitzt werden. Alternativ können auch Verbindungen der Formel (III), z.B. im Falle verwendeter Hydroxyverbindungen, zugleich Verdünnungsmittel sein.

Die Reaktionsdauer beträgt ca. 2 bis 48 Stunden. Die Umsetzung erfolgt bei Temperaturen zwischen +10 °C und +200 °C, bevorzugt zwischen +20 °C und +150 °C, besonders bevorzugt bei Raumtemperatur oder Siedetemperatur des verwendeten Verdünnungsmittels. Vorzugsweise arbeitet man bei dem Druck, der sich beim Erhitzen auf die erforderliche Reaktionstemperatur unter den Reaktionsbedingungen einstellt.

Zur Durchführung des erfindungsgemäßen Verfahrens setzt man pro Mol an 3-Aryl-5-alkyl-isoxazol-4-carbonsäurehalogenid der Formel (II) im allgemeinen 1,0 bis 4,0 Mol, vorzugsweise 1,0 bis 2,0 Mol, an Amino-, Hydrazin-, Mercapto- oder Hydroxyverbindungen der allgemeinen Formel (III) ein. Gegebenenfalls kann auch ein Überschuß an Hydroxyverbindungen verwendet werden.

Alternativ können auch die 3-Aryl-5-alkyl-isoxazol-4-carbonsäuren der allgemeinen Formel (IX) mit Amino-, Hydrazin-, Mercapto- oder Hydroxyverbindungen der Formel (III) in Gegenwart von dehydratisierenden Mitteln, beispielsweise Dicyclohexylcarbodiimid (vgl. C.S. Nion et al. Heterocycles 24 (1986) 2, S. 401), eingesetzt werden.

Nach vollendeter Umsetzung wird das Reaktionsgemisch gegebenenfalls im Vakuum eingeengt (zu ca. 50 %), der Rückstand mit wäßriger Säure versetzt und die Verbindungen der Formel (I) in an sich bekannter Weise aufgearbeitet Die anfallenden Produkte lassen sich in üblicher Weise durch Umkristallisieren, Vakuumdestillation oder Säulenchromatographie reinigen (vgl. auch die Herstellungsbeispiele).

Die Wirkstoffe eignen sich bei günstiger Warmblütertoxität zur Bekämpfung von pathogenen Endoparasiten, die bei Menschen und in der Tierhaltung und Tierzucht bei Nutz-, Zucht- , Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit Todesfalle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist Zu den pathogenen Endoparasiten zählen Cestoden, Trematoden, Hematoden, insbesondere:
Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thsysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatoropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicoaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Spirurida z.B.: Gnathostoma spp., Physaloptera spp., Thelazia spp., Gongylonema spp., Habronema spp., Parabonema spp., Draschia spp., Diacunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchia z.B.: Filicollis spp., Moniliformis spp., Macracanthorhynchus spp., Prothenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten Süß- und Salzwasserfische wie z.B. Forellen, Karpfen, Aale, Reptilien, Insekten wie z.B. Honigbiene und Seidenraupe.

Zu Labor- und Versuchtieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungvorrichtungen.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; halbfeste Zubereitungen;

Formulierungen bei denen der Wirkstoff in einer Salbengrundlage oder in einer Öl in Wasser oder Wasser in Öl Emulsionsgrundlage verarbeitet ist;

feste Zubereitungen wie Pulfer, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt:

Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt:

Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt` aufgespüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit so viel Verdickungsmittel versetzt werden, daß eine klare Masse mit salbenartiger Konsistenz entsteht Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt:

Wasser, Alkanole, Glykole, Polyethylenglykole, Polypropylenglykole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butyl-ether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metasulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter, eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a..

Fettalkohole wie Isotridecylalkohol, 2-Octyldodecanol, Cetylstearyl-alkohol, Oleyl-alkohol.

Fettsäuren wie z.B. Ölsäure und ihre Gemische.

Als hydrophile Phase seien genannt:

Wasser, Alkohole wie z.B. Propylenglykol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-$\beta$-iminodipropionat oder Lecithin;

anionaktive Tenside wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten

Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmitel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Hetzmittel (Dispergiermittel) seien die weiter oben angegebenen Tenside genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumoxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfordernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Praziquantel, Pyrantel, Febantel.

Anwendungsfertige Zubereitungen enthalten den Wirkstoff in Konzentrationen von 10 bis 20 Gewichtsprozent, bevorzugt von 0,1 bis 10 Gewichtsprozent.

Zubereitungen die vor Anwendung verdünnt werden, enthalten den Wirkstoff in Konzentrationen von 0,5 bis 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

**Beispiel A**

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 1 | 10 |
| 59 | 5 |
| 62 | 10 |
| 83 | 10 |
| 116 | 5 |
| 119 | 10 |
| 127 | 5 |
| 230 | 5 |

## Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 1 | 5 |
| 59 | 10 |
| 62 | 10 |
| 83 | 10 |
| 103 | 10 |
| 116 | 10 |
| 119 | 5 |
| 127 | 10 |
| 221 | 5 |
| 230 | 10 |

## Herstellungsbeispiele

### Beispiel 1

N-Oktadecyl-3-(2,6-dichlor-phenyl)-5-ethyl-isoxazol-4-carbonsäureamid

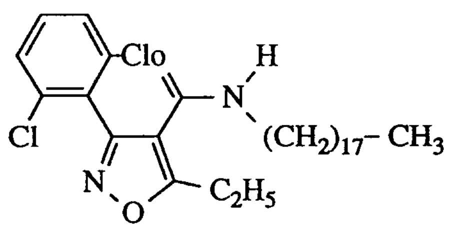

2,7 g (0,01 Mol) n-Oktadecylamin werden in 80 ml absolutem Tetrahydrofuran vorgelegt und nacheinander mit 1,5 g (0,015 Mol) Triethylamin sowie 3,05 g (0,01 Mol) 3-(2,6-Dichlor-phenyl)-5-ethyl-isoxazol-4-carbonsäurechlorid versetzt. Die Mischung wird bis zum vollständigen Umsatz (ca. 12 Stunden) bei Raumtemperatur gerührt. Danach wird der gesamte Reaktionsansatz in 400 ml 10 %ige Salzsäure eingetragen, verrührt und der ausgefallende Feststoff abgetrennt. Man erhält 5,2 g (96,8 % der Theorie) N-Oktadecyl-3-(2,6-dichlor-phenyl)-5-ethyl-isoxazol-4-carbonsäureamid.
Fp.: 55 ° C
$^1$H-NMR ($CDCl_3$):
0,88 (t, 3H, $-CH_3$; J = 7,5 Hz); 1,18-1,20 (br., 2H, $-CH_2-$); 1,26 (br.; 30H, $-(CH_2)_{15}-$); 1,41 (t, 3H, $-CH_3$; J = 7,5 Hz); 3.16-3,27 (m, 4H, 2 x $-CH_2-$; J = 7,5 Hz); 5,12 (br., 1H, NH); 7,38-7,51 (br., 3H, arom.-H)ppm

### Beispiel 2

3-(4-Chlor-phenyl)-5-methyl-isoxazol-4-carbonsäure-2-fluor-ethylester

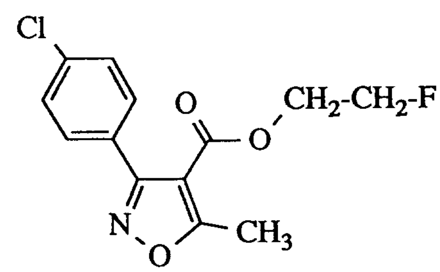

5,0 g (19,5 mMol) 3-(4-Chlor-phenyl)-5-methyl-4-isoxazol-carbonsäurechlorid werden in 10 ml 2-Fluorethanol bis zum vollständigen Umsatz (ca. 12 Stunden) bei Rückflußtemperatur gerührt. Danach wird der gesamte Reaktionsansatz in Wasser eingetragen und das abgeschiedene Öl mit Ether extrahiert. Die organische Phase wird abgetrennt, mit Wasser gewaschen, über Natriumsulfat getrocknet und anschließend das Lösungsmittel abdestilliert. Man erhält 4,0 g (72,3 % der Theorie) 3-(4-Chlor-phenyl)-5-methyl-isoxazol-4-carbonsäure-2-fluor-ethylester.
Fp.: 62 ° C
$^1$H-NMR ($CDCl_3$):
2,76 (s, 3H, $-CH_3$); 4,37-4,67(3m, 4H, $-CH_2-CH_2-$); 7,42; 7,59 (2d, 4H, arom.-H; J = 8,5 Hz)ppm

**Beispiel 3**

3-(2,6-Dichlor-phenyl)-5-methyl-isoxazol-4-carbonsäure-thiomorpholid

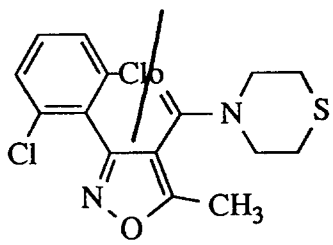

1,8 g (17,2 mMol) Thiomorpholin werden in 60 mi absolutem Tetrahydrofuran vorgelegt und nacheinander mit 2,8 g (27,5 mMol) Triethylamin und 5,0 g (17,2 mMol) 3-(2,6-Dichlor-phenyl)-5-methyl-isoxazol-4-carbonsäurechlorid versetzt. Das Reaktionsgemisch wird bis zum vollständigen Umsatz (ca 12 Stunden) beim Raumtemperatur gerührt. Danach wird der gesamte Reaktionsansatz auf 900 ml Wasser gegeben, der ausgefallene Feststoff abgetrent, mit Wasser nachgewaschen und im Trockenschrank bei 50 °C getrocknet. Man erhält 5,5 g (89,6 % der Theorie) 3-(2,6-Dichlor-phenyl)-5-methyl-isoxazol-4-carbonsäure-thiomorpholid.
Fp.: 129-131 °C
$^1$H-NMR ($CDCl_3$):
2,36 (br., 4H, $-CH_2-S-CH_2-$); 2,58 (s, 3H, $-CH_3$); 3.69 (br., 4H, $-CH_2-N-CH_2$); 7,34-7,46 (m, 3H, arom.-H)ppm

Beispiel 4

3-(2,6-Dichlor-phenyl)-5-methy-isoxazol-4-carbonsäure-butylthioester

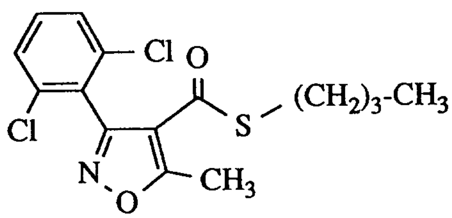

Zu einer Suspension aus 5,0 g (17,2 mMol) 3-2,6-Dichlor-phenyl)-5-methyl-isoxazol-4-carbonsäurechlorid in 60 ml absolutem Tetrahydrofuran werden bei Raumtemperatur portionsweise 1,9 g (17,2 mMol) Natrium-n-butanthiolat gegeben. Anschließend wird noch ca. 12 Stunden nachgerührt und der gesamte Reaktionsansatz im Vakuum eingeengt. Der verbleibende ölige Rückstand wird in Essigsäureethylester aufgenommen und zweimal mit Wasser ausgeschüttelt. Nach dem Trocknen und Einengen der organischen Phase erhält man 4,4 g (74,3 % der Theorie) 3-(2,6-Dichlor-phenyl)-5-methyl-isoxazol-4-carbonsäurebutylthioester
Fp.: Öl
$^1$H-NMR ($CDCl_3$): 0,87 (t, 3H, $-CH_3$; J = 7,3 Hz); 1,28-1,50(2m, 4H, $-CH_2-CH_2-$; J = 7,3 Hz); 2,82 (s, 3H, het.-$CH_3$); 2,90 (t, 2H, $-S-CH_2-$; J = 7,3 Hz); 7,35-7,45 (m, 3H, arom.-H) ppm.

Tabelle 1

Beispiele für die Verbindungen der Formel (I)

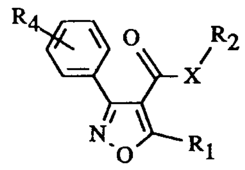

| Bsp.-Nr. | X | $R^1$ | $R^2$ | $R^4$ | Physikalische Daten |
|---|---|---|---|---|---|
| 5 | O | $CH_3$ | s-$C_4H_9$ | H | Öl |
| 6 | O | $CH_3$ | Pyrid-3-yl | H | 81°C |
| 7 | O | $CH_3$ | $CH_2$-$CH_2$-F | H | Öl |
| 8 | O | $CH_3$ | $CH_2$-$CF_3$ | H | 57°C |
| 9 | O | $CH_3$ | CH($CH_3$)-Phenyl | H | Öl |
| 10 | NH | $CH_3$ | n-$C_{18}H_{37}$ | H | 111°C |
| 11 | NH | $CH_3$ | Phenyl | H | 195-196°C |
| 12 | NH | $CH_3$ | 2,4,6-($CH_3$)$_3$-Phenyl | H | 171°C |
| 13 | NH | $CH_3$ | 2-F, 4-Cl-Phenyl | H | 149°C |
| 14 | NH | $CH_3$ | 4-$SCF_3$-Phenyl | H | 137°C |
| 15 | NH | $CH_3$ | 4-O-[($CH_2$)$_2$-O-($CH_2$)$_2$-O-$CH_3$]-Phenyl | H | 95-96°C |
| 16 | NH | $CH_3$ | Pyrid-3-yl | H | 185-190°C |
| 17 | NH | $CH_3$ | ($CH_2$)$_2$-NH-(3,4-$Cl_2$-Phenyl | H | 101-107°C |
| 18 | NH | $CH_3$ | ($CH_2$)$_3$-NH-(2-Cl-Phenyl) | H | 85-91°C |
| 19 | NH | $CH_3$ | ($CH_2$)$_2$-N($CH_3$)-Phenyl | H | 78-80°C |
| 20 | NH | $CH_3$ | 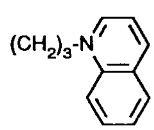 | H | 120-122°C |

Tabelle 1

(Fortsetzung)

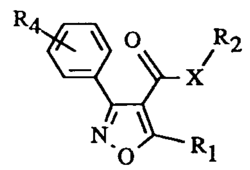

| Bsp.-Nr. | X | $R^1$ | $R^2$ | $R^4$ | Physikalische Daten |
|---|---|---|---|---|---|
| 21 | O | $CH_3$ | n-$C_{16}H_{33}$ | 2-Cl | 38-39°C |
| 22 | NH | $CH_3$ | i-$C_3H_7$ | 2-Cl | 139-140°C |
| 23 | NH | $CH_3$ | t-$C_4H_9$ | 2-Cl | 82-83°C |
| 24 | NH | $CH_3$ | c-$C_6H_{11}$ | 2-Cl | 148-149°C |
| 25 | NH | $CH_3$ | $CH_2$-(2-Cl-Phenyl) | 2-Cl | 104-105°C |
| 26 | NH | $CH_3$ | Phenyl | 2-Cl | 180-181°C |
| 27 | NH | $CH_3$ | 4-Cl-Phenyl | 2-Cl | 130-131°C |
| 28 | NH | $CH_3$ | 2-$OCF_3$-Phenyl | 2-Cl | 63-64°C |
| 29 | NH | $CH_3$ | 2-$CH_3$-Phenyl | 2-Cl | 160-161°C |
| 30 | NH | $CH_3$ | 4-$CH_3$-Phenyl | 2-Cl | 128-129°C |
| 31 | NH | $CH_3$ | 3-$CF_3$-Phenyl | 2-Cl | 118-119°C |
| 32 | NH | $CH_3$ | 4-O-(4-Cl-Phenyl)-Phenyl | 2-Cl | 157-158°C |
| 33 | NH | $CH_3$ | 4-Br-Phenyl | 2-Cl | 128-129°C |
| 34 | NH | $CH_3$ | 4-$OCH_3$-Phenyl | 2-Cl | 128-129°C |
| 35 | NH | $CH_3$ | 2-$OCH_3$,4-$NO_2$-Phenyl | 2-Cl | 142-143°C |
| 36 | NH | $CH_3$ | 2-F, 4-Cl-Phenyl | 2-Cl | 121-122°C |

Tabelle 1

(Fortsetzung)

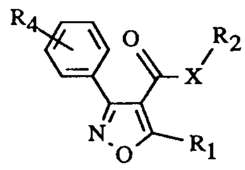

| Bsp.-Nr. | X | $R^1$ | $R^2$ | $R^4$ | Physikalische Daten |
|---|---|---|---|---|---|
| 37 | NH | $CH_3$ | 4-$SCF_3$-Phenyl | 2-Cl | 95-96°C |
| 38 | NH-NH | $CH_3$ | 3-Cl, 4-F-Phenyl | 2-Cl | 170-171°C |
| 39 | NH-NH | $CH_3$ | 2,5-$Cl_2$-Phenyl | 2-Cl | 167-168°C |
| 40 | NH-NH | $CH_3$ | 4-F-Phenyl | 2-Cl | 152-153°C |
| 41 | NH-NH | $CH_3$ | 2-$CH_3$-Phenyl | 2-Cl | 58-59°C |
| 42 | NH-NH | $CH_3$ | 2,4-$(CH_3)_2$-Phenyl | 2-Cl | 114-115°C |
| 43 | NH-NH | $CH_3$ | 2,3-$(CH_3)_2$-Phenyl | 2-Cl | 131-132°C |
| 44 | NH-NH | $CH_3$ | 3,5-$(CH_3)_2$-Phenyl | 2-Cl | 124-125°C |
| 45 | O | $CH_3$ | $CH_2$-$CF_3$ | 4-Cl | 57°C |
| 46 | O | $CH_3$ | s-$C_4H_9$ | 4-Cl | Öl |
| 47 | NH | $CH_3$ | 3-$CH_3$-Phenyl | 4-Cl | 144°C |
| 48 | NH | $CH_3$ | 2,3-$(CH_3)$-Phenyl | 4-Cl | 165°C |
| 49 | NH | $CH_3$ | 2-Cl-Phenyl | 4-Cl | 133°C |
| 50 | NH | $CH_3$ | 4-$CF_3$-Phenyl | 4-Cl | 235°C |
| 51 | NH | $CH_3$ | 2-$OCH_3$-Phenyl | 4-Cl | 119°C |
| 52 | NH | $CH_3$ | 4-$SCF_3$-Phenyl | 4-Cl | 190°C |

Tabelle 1

(Fortsetzung)

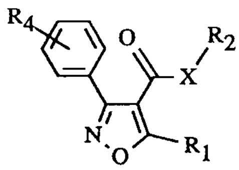

| Bsp.-Nr. | X | $R^1$ | $R^2$ | $R^4$ | Physikalische Daten |
|---|---|---|---|---|---|
| 53 | NH | $CH_3$ | Phenyl | 4-Cl | 177°C |
| 54 | O | $CH_3$ | $CH_3$ | 2,6-$Cl_2$ | 111-112°C |
| 55 | O | $C_2H_5$ | $CH_3$ | 2,6-$Cl_2$ | 58°C |
| 56 | O | $C_2H_5$ | $C_2H_5$ | 2,6-$Cl_2$ | 63°C |
| 57 | O | $CH_3$ | n-$C_{16}H_{33}$ | 2,6-$Cl_2$ | 353-354°C |
| 58 | O | $CH_3$ | Phenyl | 2,6-$Cl_2$ | 70-71°C |
| 59 | O | $CH_3$ | 4-$CH_3$-Phenyl | 2,6-$Cl_2$ | 96-97°C |
| 60 | O | $CH_3$ | 2-$CH_3$-Phenyl | 2,6-$Cl_2$ | 108-109°C |
| 61 | O | $CH_3$ | 4-$OCH_3$-Phenyl | 2,6-$Cl_2$ | 68-69°C |
| 62 | O | $CH_3$ | 4-Cl-Phenyl | 2,6-$Cl_2$ | 112-113°C |
| 63 | S | $CH_3$ | $CH_3$ | 2,6-$Cl_2$ | 83- 84°C |
| 64 | S | $CH_3$ | i-$C_3H_7$ | 2,6-$Cl_2$ | Öl |
| 65 | S | $CH_3$ | 4-F-Phenyl | 2,6-$Cl_2$ | 104-105°C |
| 66 | S | $CH_3$ | 4-$CH_3$-Phenyl | 2,6-$Cl_2$ | Öl |
| 67 | NH | $CH_3$ | n-$C_3H_7$ | 2,6-$Cl_2$ | 137-141°C |
| 68 | NH | $CH_3$ | i-$C_3H_7$ | 2,6-$Cl_2$ | 158-159°C |
| 69 | NH | $CH_3$ | n-$C_4H_9$ | 2,6-$Cl_2$ | 92-93°C |
| 70 | NH | $CH_3$ | i-$C_4H_9$ | 2,6-$Cl_2$ | 111-117°C |
| 71 | NH | $CH_3$ | s-$C_4H_9$ | 2,6-$Cl_2$ | 154-155°C |
| 72 | NH | $CH_3$ | t-$C_4H_9$ | 2,6-$Cl_2$ | 117-118°C |

Tabelle 1

(Fortsetzung)

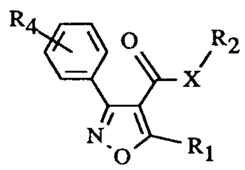

| Bsp.-Nr. | X | $R^1$ | $R^2$ | $R^4$ | Physikalische Daten |
|---|---|---|---|---|---|
| 73 | NH | $CH_3$ | $C(CH_3)_2$-$C_2H_5$ | 2,6-$Cl_2$ | 110-116°C |
| 74 | NH | $CH_3$ | $CH_2$-$C(CH_3)_3$ | 2,6-$Cl_2$ | 152-156°C |
| 75 | NH | $CH_3$ | n-$C_6H_{13}$ | 2,6-$Cl_2$ | Öl |
| 76 | NH | $CH_3$ | c-$C_6H_{11}$ | 2,6-$Cl_2$ | 117-118°C |
| 77 | NH | $CH_3$ | $CH_2$-c-$C_6H_{11}$ | 2,6-$Cl_2$ | 82-84°C |
| 78 | NH | $CH_3$ | c-$C_7H_{13}$ | 2,6-$Cl_2$ | 78-85°C |
| 79 | NH | $CH_3$ | $CH_2$-$CH(C_2H_5)$-$C_4H_9$ | 2,6-$Cl_2$ | Öl |
| 80 | NH | $CH_3$ | n-$C_{12}H_{25}$ | 2,6-$Cl_2$ | 50-56°C |
| 81 | NH | $CH_3$ | n-$C_{14}$-$H_{29}$ | 2,6-$Cl_2$ | 54-57°C |
| 82 | NH | $CH_3$ | n-$C_{16}H_{33}$ | 2,6-$Cl_2$ | 58-62°C |
| 83 | NH | $CH_3$ | n-$C_{18}H_{37}$ | 2,6-$Cl_2$ | 67-73°C |
| 84 | NH | $CH_3$ | n-$C_{19}H_{39}$ | 2,6-$Cl_2$ | 58-60°C |
| 85 | NH | $CH_3$ | $CH_2$-Phenyl | 2,6-$Cl_2$ | 104-108°C |
| 86 | NH | $CH_3$ | $CH_2$-(4-$SO_2$-$NH_2$-Phenyl) | 2,6-$Cl_2$ | 207-222°C |
| 87 | NH | $CH_3$ | $CH_2$-(2-Cl-Phenyl) | 2,6-$Cl_2$ | 95-98°C |

Tabelle 1

(Fortsetzung)

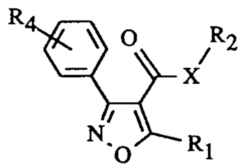

| Bsp.-Nr. | X | $R^1$ | $R^2$ | $R^4$ | Physikalische Daten |
|---|---|---|---|---|---|
| 88 | NH | $CH_3$ | $CH_2$-Pyrid-2-yl | 2,6-$Cl_2$ | 124-156°C |
| 89 | NH | $CH_3$ | $CH(CH_3)$-Phenyl | 2,6-$Cl_2$ | Öl |
| 90 | NH | $CH_3$ | $(CH_2)_2$-S-$C(CH_3)_3$ | 2,6-$Cl_2$ | 100-102°C |
| 91 | NH | $CH_3$ | $(CH_2)_2$-NH-(3,4-$Cl_2$-Phenyl) | 2,6-$Cl_2$ | 221-224°C |
| 92 | NH | $CH_3$ | $(CH_2)_3$-NH-(2-Cl-Phenyl) | 2,6-$Cl_2$ | 134-138°C |
| 93 | NH | $CH_3$ | Phenyl | 2,6-$Cl_2$ | 147°C |
| 94 | NH | $CH_3$ | 2-Cl-Phenyl | 2,6-$Cl_2$ | 172°C |
| 95 | NH | $C_2H_5$ | 2-Cl-Phenyl | 2,6-$Cl_2$ | 118°C |
| 96 | NH | $CH_3$ | 3-Cl-Phenyl | 2,6-$Cl_2$ | 163-166°C |
| 97 | NH | $CH_3$ | 4-Cl-Phenyl | 2,6-$Cl_2$ | 183°C |
| 98 | NH | $C_2H_5$ | 4-Cl-Phenyl | 2,6-$Cl_2$ | 138°C |
| 99 | NH | $CH_3$ | 3,5-$Cl_2$-Phenyl | 2,6-$Cl_2$ | 160-170°C |
| 100 | NH | $CH_3$ | 3,4-$Cl_2$-Phenyl | 2,6-$Cl_2$ | 190-196°C |
| 101 | NH | $CH_3$ | 2,6-$Cl_2$-Phenyl | 2,6-$Cl_2$ | 148-152°C |
| 102 | NH | $CH_3$ | 4-F-Phenyl | 2,6-$Cl_2$ | 149-152°C |
| 103 | NH | $CH_3$ | 3,5-$F_2$-Phenyl | 2,6-$Cl_2$ | 148-156°C |

Tabelle 1

(Fortsetzung)

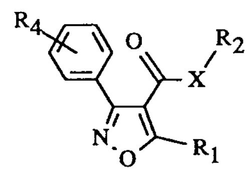

| Bsp.-Nr. | X | $R^1$ | $R^2$ | $R^4$ | Physikalische Daten |
|---|---|---|---|---|---|
| 104 | NH | $CH_3$ | 2,4-$F_2$-Phenyl | 2,6-$Cl_2$ | 149-157°C |
| 105 | NH | $CH_3$ | 2-F,4-Cl-Phenyl | 2,6-$Cl_2$ | 144-156°C |
| 106 | NH | $C_2H_5$ | 3,5-$F_2$-Phenyl | 2,6-$Cl_2$ | 139°C |
| 107 | NH | $C_2H_5$ | 2-F, 6-Cl-Phenyl | 2,6-$Cl_2$ | 116°C |
| 108 | NH | $CH_3$ | 4-Br-Phenyl | 2,6-$Cl_2$ | 189-191°C |
| 109 | NH | $C_2H_5$ | 4-Br-Phenyl | 2,6-$Cl_2$ | 145°C |
| 110 | NH | $CH_3$ | 4-$OCH_3$-Phenyl | 2,6-$Cl_2$ | 123-125°C |
| 111 | NH | $CH_3$ | 3-$OCH_3$-Phenyl | 2,6-$Cl_2$ | 176-178°C |
| 112 | NH | $C_2H_5$ | 2-$OCH_3$-Phenyl | 2,6-$Cl_2$ | 115°C |
| 113 | NH | $CH_3$ | 2-$OCH_3$, 5-$NO_2$-Phenyl | 2,6-$Cl_2$ | 199-201°C |
| 114 | NH | $CH_3$ | 4-O-[$(CH_2)_2$-O-$(CH_2)_2$-O-$CH_3$]-Phenyl | 2,6-$Cl_2$ | Öl |
| 115 | NH | $CH_3$ | 4-O-(4-Cl-Phenyl)-phenyl | 2,6-$Cl_2$ | 136-139°C |
| 116 | NH | $CH_3$ | 2-$CH_3$-Phenyl | 2,6-$Cl_2$ | 138-139°C |
| 117 | NH | $C_2H_5$ | 2-$CH_3$-Phenyl | 2,6-$Cl_2$ | 94°C |
| 118 | NH | $C_2H_5$ | 3-$CH_3$-Phenyl | 2,6-$Cl_2$ | 111°C |
| 119 | NH | $CH_3$ | 4-$CH_3$-Phenyl | 2,6-$Cl_2$ | 153-154°C |

Tabelle 1

(Fortsetzung)

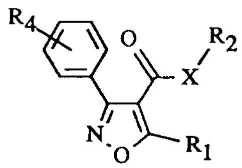

| Bsp.-Nr. | X | $R^1$ | $R^2$ | $R^4$ | Physikalische Daten |
|---|---|---|---|---|---|
| 120 | NH | $C_2H_5$ | 2,3-$(CH_3)$-Phenyl | 2,6-$Cl_2$ | 123°C |
| 121 | NH | $CH_3$ | 2,6-$(CH_3)_2$-Phenyl | 2,6-$Cl_2$ | 160-163°C |
| 122 | NH | $C_2H_5$ | 2,4,6-$(CH_3)_3$-Phenyl | 2,6-$Cl_2$ | 142°C |
| 123 | NH | $CH_3$ | 2-$CH_3$, 3-Cl-Phenyl | 2,6-$Cl_2$ | 125-126°C |
| 124 | NH | $CH_3$ | 3-Cl, 4-$CH_3$-Phenyl | 2,6-$Cl_2$ | 164-170°C |
| 125 | NH | $CH_3$ | 2-$CH_3$, 4-$NO_2$-Phenyl | 2,6-$Cl_2$ | 170-178°C |
| 126 | NH | $CH_3$ | 3-$CF_3$-Phenyl | 2,6-$Cl_2$ | 164°C |
| 127 | NH | $C_2H_5$ | 4-$SCF_3$-Phenyl | 2,6-$Cl_2$ | 103°C |
| 128 | NH | $C_2H_5$ | 4-$SCF_3$-Phenyl | 2,6-$Cl_2$ | 86°C |
| 129 | NH | $CH_3$ | 3-$SCH_3$-Phenyl | 2,6-$Cl_2$ | 199-203°C |
| 130 | NH | $CH_3$ | 4-$CF_3$-Phenyl | 2,6-$Cl_2$ | 136°C |
| 131 | NH | $CH_3$ | 2-$OCF_3$-Phenyl | 2,6-$Cl_2$ | 106-107°C |
| 132 | NH | $CH_3$ | 2-$NO_2$-Phenyl | 2,6-$Cl_2$ | 151-153°C |
| 133 | NH | $CH_3$ | 2-SH-Phenyl | 2,6-$Cl_2$ | 196-199°C |
| 134 | NH | $CH_3$ | 4-OH, 3-Cl-Phenyl | 2,6-$Cl_2$ | 110-114°C |
| 135 | NH | $CH_3$ | 6-O-[4-Cl, 3,5-$(CH_3)_2$-Phenyl]-pyrid-3-yl | 2,6-$Cl_2$ | 177-181°C |

Tabelle 1

(Fortsetzung)

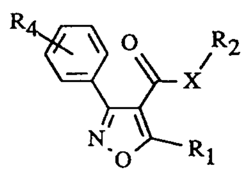

| Bsp.-Nr. | X | $R^1$ | $R^2$ | $R^4$ | Physikalische Daten |
|---|---|---|---|---|---|
| 136 | NH | $CH_3$ | 6-O-[3-C($CH_3$)$_3$-Phenyl]-pyrid-3-yl | 2,6-$Cl_2$ | 159-160°C |
| 137 | NH | $CH_3$ | 6-O-(3-$CF_3$-Phenyl)-pyrid-3-yl | 2,6-$Cl_2$ | 132-135°C |
| 138 | NH | $CH_3$ | 6-O-(2,6-$Cl_2$-Phenyl)-pyrid-3-yl | 2,6-$Cl_2$ | 162-167°C |
| 139 | NH | $CH_3$ | 6-O-(3-Cl-Phenyl)-pyrid-3-yl | 2,6-$Cl_2$ | 74-95°C |
| 140 | NH | $CH_3$ | 6-O-(4-Br-Phenyl)-pyrid-3-yl | 2,6-$Cl_2$ | 169-173°C |
| 141 | NH | $CH_3$ | 6-O-(4-Cl-Phenyl)-pyrid-3-yl | 2,6-$Cl_2$ | 176-180°C |
| 142 | NH | $CH_3$ | Pyrimid-2-yl | 2,6-$Cl_2$ | > 225°C |
| 143 | NH | $C_2H_5$ | 6-$CH_3$-Pyrimid-2-yl | 2,6-$Cl_2$ | 177°C Zers. |
| 144 | -N- | $CH_3$ | -$CH_2$-CH($CH_3$)-O-CH($CH_3$)-$CH_2$- | 2,6-$Cl_2$ | 171-173°C |
| 145 | -N- | $CH_3$ | -$(CH_2)_4$- | 2,6-$Cl_2$ | 163-164°C |
| 146 | -N- | $CH_3$ | -$(CH_2)_5$- | 2,6-$Cl_2$ | 127-128°C |
| 147 | -N-$CH_3$ | $CH_3$ | $C_{18}H_{37}$ | 2,6-$Cl_2$ | 34- 36°C |
| 148 | -N-$C_{12}H_{25}$ | $CH_3$ | $C_{14}H_{29}$ | 2,6-$Cl_2$ | Öl |
| 149 | -N-$C_{12}H_{25}$ | $CH_3$ | $C_{16}H_{33}$ | 2,6-$Cl_2$ | Öl |
| 150 | -N-$C_{12}H_{25}$ | $CH_3$ | $C_{18}H_{37}$ | 2,6-$Cl_2$ | Öl |
| 151 | -N-$C_{14}H_{29}$ | $CH_3$ | $C_{14}H_{29}$ | 2,6-$Cl_2$ | Öl |

Tabelle 1

(Fortsetzung)

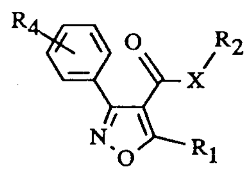

| Bsp.-Nr. | X | $R^1$ | $R^2$ | $R^4$ | Physikalische Daten |
|---|---|---|---|---|---|
| 152 | $-N-C_{14}H_{29}$ | $CH_3$ | $C_{16}H_{33}$ | 2,6-$Cl_2$ | Öl |
| 153 | $-N-C_{16}H_{33}$ | $CH_3$ | $(CH_2)_8-CH=CH-C_8H_{17}$ | 2,6-$Cl_2$ | Öl |
| 154 | $-N-C_{18}H_{37}$ | $CH_3$ | $(CH_2)_8-CH=CH-C_8H_{17}$ | 2,6-$Cl_2$ | Öl |
| 155 | -N- | $CH_3$ | $-(CH_2)_2-N(CH_2$-Phenyl)$-(CH_2)_2-$ | 2,6-$Cl_2$ | 104-106°C |
| 156 | -N- | $CH_3$ | $-(CH_2)_2-N[2,6-(CH_3)_2-$ Phenyl]$-(CH_2)_2-$ | 2,6-$Cl_2$ | 227-228°C |
| 157 | -N- | $CH_3$ | $-(CH_2)_2-N(2$-Cl-Phenyl]$-(CH_2)_2-$ | 2,6-$Cl_2$ | 170-171°C |
| 158 | -N- | $CH_3$ | $-(CH_2)_2-N(3-CF_3$, 4-Cl-Phenyl)$-(CH_2)_2-$ | 2,6-$Cl_2$ | 227-228°C |
| 159 | NH-NH | $CH_3$ | 3,4-$(CH_3)_2$-Phenyl | 2,6-$Cl_2$ | 162-163°C |
| 160 | NH-NH | $CH_3$ | 2,6-$Cl_2$, 3,5-$F_2$, 4-$CF_3$-Phenyl | 2,6-$Cl_2$ | 102-103°C |
| 161 | NH-NH | $CH_3$ | 2,3,6-$Cl_3$, 4-$CF_3$-Phenyl | 2,6-$Cl_2$ | 122-123°C |
| 162 | NH-NH | $CH_3$ | 2,3,5-$F_3$, 4,6-$Cl_2$-Phenyl | 2,6-$Cl_2$ | 158-159°C |
| 163 | NH-NH | $CH_3$ | 2,6-$Cl_2$, 4-$CF_3$-Phenyl | 2,6-$Cl_2$ | 162-163°C |

Tabelle 1

(Fortsetzung)

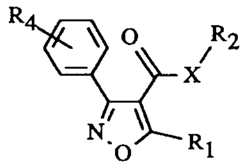

| Bsp.-Nr. | X | $R^1$ | $R^2$ | $R^4$ | Physikalische Daten |
|---|---|---|---|---|---|
| 164 | NH-NH | $CH_3$ | 4-Cl-Phenyl | 2,6-$Cl_2$ | 160-161°C |
| 165 | NH-NH | $CH_3$ | 2,6-$Cl_2$, 4-$SO_2$-$OCF_3$-Phenyl | 2,6-$Cl_2$ | 152-153°C |
| 166 | NH-NH | $CH_3$ | 2,6-$Cl_2$-Phenyl | 2,6-$Cl_2$ | 148-149°C |
| 167 | NH-NH | $CH_3$ | 2,5-$Cl_2$-Phenyl | 2,6-$Cl_2$ | 188-189°C |
| 168 | NH-NH | $CH_3$ | 2,5-$(CH_3)_2$-Phenyl | 2,6-$Cl_2$ | 164-165°C |
| 169 | NH-NH | $CH_3$ | 4-$CH_3$-Phenyl | 2,6-$Cl_2$ | 83-89°C |
| 170 | NH-NH | $CH_3$ | 3,5-$(CH_3)_2$-Phenyl | 2,6-$Cl_2$ | 168-169°C |
| 171 | NH-NH | $CH_3$ | 3-Cl, 4-F-Phenyl | 2,6-$Cl_2$ | 201-202°C |
| 172 | NH-NH | $CH_3$ | 3-Cl, 5-$CF_3$-Pyrid-2-yl | 2,6-$Cl_2$ | 62-63°C |
| 173 | NH-NH | $CH_3$ | 5-Cl, 3-$CF_3$-Pyrid-2-yl | 2,6-$Cl_2$ | 105-106°C |
| 174 | NH-NH | $CH_3$ | 3,5-$(CH_3)_2$-Phenyl | 2,6-$Cl_2$ | 158-159°C |
| 175 | O | $CH_3$ | $CH_3$ | 2-F, 6-Cl | 38-39°C |
| 176 | O | $CH_3$ | 4-$CH_3$-Phenyl | 2-F, 6-Cl | 73-74°C |
| 177 | O | $CH_3$ | 4-Cl-Phenyl | 2-F, 6-Cl | 72-73°C |
| 178 | NH | $CH_3$ | n-$C_3H_7$ | 2-F, 6-Cl | 106-109°C |
| 179 | NH | $CH_3$ | i-$C_3H_7$ | 2-F, 6-Cl | 134-135°C |

Tabelle 1

(Fortsetzung)

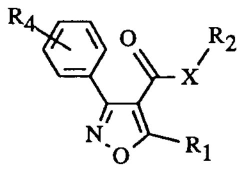

| Bsp.-Nr. | X | $R^1$ | $R^2$ | $R^4$ | Physikalische Daten |
|---|---|---|---|---|---|
| 180 | NH | $CH_3$ | n-$C_4H_9$ | 2-F, 6-Cl | 134-135°C |
| 181 | NH | $CH_3$ | t-$C_4H_9$ | 2-F, 6-Cl | 58-59°C |
| 182 | NH | $CH_3$ | s-$C_4H_9$ | 2-F, 6-Cl | 136-138°C |
| 183 | NH | $CH_3$ | i-$C_4H_9$ | 2-F, 6-Cl | 114-120°C |
| 184 | NH | $CH_3$ | $C(CH_3)_2$-$C_2H_5$ | 2-F, 6-Cl | 114-124°C |
| 185 | NH | $CH_3$ | n-$C_6H_{13}$ | 2-F, 6-Cl | 60-64°C |
| 186 | NH | $CH_3$ | c-$C_6H_{11}$ | 2-F, 6-Cl | 118-119°C |
| 187 | NH | $CH_3$ | $CH_2$-$CH(C_4H_9)$-$C_2H_5$ | 2-F, 6-Cl | Öl |
| 188 | NH | $CH_3$ | c-$C_5H_{11}$ | 2-F, 6-Cl | 66-67°C |
| 189 | NH | $CH_3$ | c-$C_7H_{13}$ | 2-F, 6-Cl | 77-90°C |
| 190 | NH | $CH_3$ | n-$C_{12}H_{25}$ | 2-F, 6-Cl | 87-90°C |
| 191 | NH | $CH_3$ | n-$C_{14}H_{29}$ | 2-F, 6-Cl | 65-75°C |
| 192 | NH | $CH_3$ | n-$C_{16}H_{33}$ | 2-F, 6-Cl | 66-70°C |
| 193 | NH | $CH_3$ | n-$C_{18}H_{37}$ | 2-F, 6-Cl | 77-80°C |
| 194 | NH | $CH_3$ | $CH(CH_3)$-Phenyl | 2-F, 6-Cl | Öl |
| 195 | NH | $CH_3$ | $CH_2$-$C(CH_3)_3$ | 2-F, 6-Cl | 134°C |

Tabelle 1

(Fortsetzung)

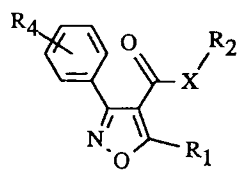

| Bsp.-Nr. | X | $R^1$ | $R^2$ | $R^4$ | Physikalische Daten |
|---|---|---|---|---|---|
| 196 | NH | $CH_3$ | $CH_2$-c-$C_6H_{11}$ | 2-F, 6-Cl | 60-74°C |
| 197 | NH | $CH_3$ | $CH_2$-Phenyl | 2-F, 6-Cl | 116-118°C |
| 198 | NH | $CH_3$ | $CH_2$-(2-Cl-Phenyl) | 2-F, 6-Cl | 118-121°C |
| 199 | NH | $CH_3$ | $CH_2$-(4-$SO_2$-$NH_2$-Phenyl) | 2-F, 6-Cl | 199-206°C |
| 200 | NH | $CH_3$ | $CH_2$-Pyrid-2-yl | 2-F, 6-Cl | 136-138°C |
| 201 | NH | $CH_3$ | $(CH_2)_3$- 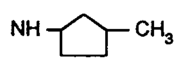 | 2-F, 6-Cl | Öl |
| 202 | NH | $CH_3$ | 4-OH, 3-Cl-Phenyl | 2-F, 6-Cl | 170-175°C |
| 203 | NH | $CH_3$ | 2-$OCH_3$, 5-$NO_2$-Phenyl | 2-F, 6-Cl | 184-186°C |
| 204 | NH | $CH_3$ | 3-Cl, 4-$CH_3$-Phenyl | 2-F, 6-Cl | 118°C |
| 205 | NH | $CH_3$ | 2-$NO_2$-Phenyl | 2-F, 6-Cl | 60-64°C |
| 206 | NH | $CH_3$ | 6-O-[4-Cl, 3,5-$(CH_3)_2$-Phenyl]-pyrid-3-yl | 2-F, 6-Cl | 165-170°C |
| 207 | NH | $CH_3$ | 6-O-(4-Br-Phenyl)-pyrid-3-yl | 2-F, 6-Cl | 142-146°C |
| 208 | NH | $CH_3$ | 6-O-(2,6-$Cl_2$-Phenyl)-pyrid-3-yl | 2-F, 6-Cl | 130-140°C |
| 209 | NH | $CH_3$ | 6-O-(3-t-$C_4H_9$-Phenyl)-pyrid-3-yl | 2-F, 6-Cl | 136-140°C |
| 210 | NH | $CH_3$ | 2,4-$F_2$-Phenyl | 2-F, 6-Cl | 134-137°C |

Tabelle 1

(Fortsetzung)

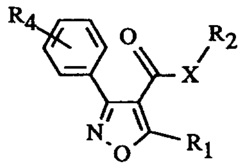

| Bsp.-Nr. | X | $R^1$ | $R^2$ | $R^4$ | Physikalische Daten |
|---|---|---|---|---|---|
| 211 | NH | $CH_3$ | 6-O-(3-$CF_3$-Phenyl)-pyrid-3-yl | 2-F, 6-Cl | 188-190°C |
| 212 | NH | $CH_3$ | 6-O-(3-Cl-Phenyl)-pyrid-3-yl | 2-F, 6-Cl | 134-139°C |
| 213 | NH | $CH_3$ | 6-O-(4-Cl-Phenyl)-pyrid-3-yl | 2-F, 6-Cl | 130°C |
| 214 | NH | $CH_3$ | Pyrid-3-yl | 2-F, 6-Cl | > 89°C Zers. |
| 215 | NH | $CH_3$ | $(CH_2)_3$-NH-(2-Cl-Phenyl) | 2-F, 6-Cl | 140-142°C |
| 216 | NH | $CH_3$ | $(CH_2)_2$-NH-(3,4-$Cl_2$-Phenyl) | 2-F, 6-Cl | 195-198°C |
| 217 | NH | $CH_3$ | Phenyl | 2-F, 6-Cl | 172-173°C |
| 218 | NH | $CH_3$ | 2-Cl-Phenyl | 2-F, 6-Cl | 123-124°C |
| 219 | NH | $CH_3$ | 3-Cl-Phenyl | 2-F, 6-Cl | 154-160°C |

Tabelle 1

(Fortsetzung)

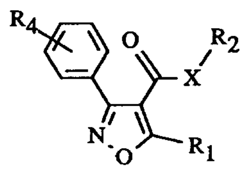

| Bsp.-Nr. | X | $R^1$ | $R^2$ | $R^4$ | Physikalische Daten |
|---|---|---|---|---|---|
| 220 | NH | $CH_3$ | 4-Cl-Phenyl | 2-F, 6-Cl | 133-134°C |
| 221 | NH | $CH_3$ | 2-$CH_3$-Phenyl | 2-F, 6-Cl | 126-127°C |
| 222 | NH | $CH_3$ | 4-$CH_3$-Phenyl | 2-F, 6-Cl | 76-77°C |
| 223 | NH | $CH_3$ | 3,4-$Cl_2$-Phenyl | 2-F, 6-Cl | 154-158°C |
| 224 | NH | $CH_3$ | 3,5-$Cl_2$-Phenyl | 2-F, 6-Cl | 180-184°C |
| 225 | NH | $CH_3$ | 2,4,6-$Cl_2$-Phenyl | 2-F, 6-Cl | 224°C |
| 226 | NH | $CH_3$ | 4-$CF_3$-Phenyl | 2-F, 6-Cl | 92-115°C |
| 227 | NH | $CH_3$ | 3-$CF_3$-Phenyl | 2-F, 6-Cl | 126-128°C |
| 228 | NH | $CH_3$ | 4-F-Phenyl | 2-F, 6-Cl | 116-119°C |
| 229 | NH | $CH_3$ | 3,5-$F_2$-Phenyl | 2-F, 6-Cl | 138-140°C |
| 230 | NH | $CH_3$ | 2-F, 4-Cl-Phenyl | 2-F, 6-Cl | 118-156°C |
| 231 | NH | $CH_3$ | 4-Br-Phenyl | 2-F, 6-Cl | 187-188°C |
| 232 | NH | $CH_3$ | 3-$OCH_3$-Phenyl | 2-F, 6-Cl | 121-125°C |
| 233 | NH | $CH_3$ | 3-$SCH_3$-Phenyl | 2-F, 6-Cl | 155-159°C |
| 234 | NH | $CH_3$ | SH-Phenyl | 2-F, 6-Cl | 172-177° C |
| 235 | NH | $CH_3$ | 4-$OCH_3$-Phenyl | 2-F, 6-Cl | 125-126°C |

Tabelle 1

(Fortsetzung)

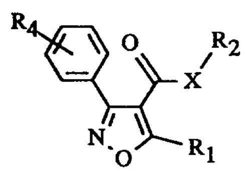

| Bsp.-Nr. | X | $R^1$ | $R^2$ | $R^4$ | Physikalische Daten |
|---|---|---|---|---|---|
| 236 | NH | $CH_3$ | 4-O-[($CH_2$)$_2$-O-($CH_2$)$_2$-$OCH_3$]-Phenyl | 2-F, 6-Cl | 90-98°C |
| 237 | NH | $CH_3$ | 4-O-(4-Cl-Phenyl)-phenyl | 2-F, 6-Cl | 130-134°C |
| 238 | NH | $CH_3$ | 2,6-($CH_3$)$_2$-Phenyl | 2-F, 6-Cl | 194-195°C |
| 239 | NH-NH | $CH_3$ | 2,6-$Cl_2$,3,5-$F_2$,4-$CF_3$-Phenyl | 2-F, 6-Cl | 119-120°C |
| 240 | NH-NH | $CH_3$ | 2,3,6-$Cl_3$,4-$CF_3$-Phenyl | 2-F, 6-Cl | 130-131°C |
| 241 | NH-NH | $CH_3$ | 2,6-$Cl_2$, 4-$CF_3$-Phenyl | 2-F, 6-Cl | 160-161°C |
| 242 | NH-NH | $CH_3$ | 2,6-$Cl_2$, 4-$SO_2$-$OCF_3$-Phenyl | 2-F, 6-Cl | 141-142°C |
| 243 | NH-NH | $CH_3$ | 3-Cl,4-F-Phenyl | 2-F, 6-Cl | 121-122°C |
| 244 | NH-NH | $CH_3$ | 2,4-$F_2$-Phenyl | 2-F, 6-Cl | 174-175°C |
| 245 | NH-NH | $CH_3$ | 3-Cl, 5-$CF_3$-Pyrid-2-yl | 2-F, 6-Cl | 112-113°C |
| 246 | NH-NH | $CH_3$ | 5-Cl, 3-$CF_3$-Pyrid-2-yl | 2-F, 6-Cl | 118-119°C |
| 247 | NH-NH | $CH_3$ | 4-$CH_3$-Phenyl | 2-F, 6-Cl | 62-63°C |
| 248 | NH-NH | $CH_3$ | 2,4-($CH_3$)$_2$-Phenyl | 2-F, 6-Cl | 158-159°C |
| 249 | NH-NH | $CH_3$ | 2,5-($CH_3$)$_2$-Phenyl | 2-F, 6-Cl | 155-156°C |
| 250 | NH-NH | $CH_3$ | 3,4-($CH_3$)$_2$-Phenyl | 2-F, 6-Cl | 118-119°C |
| 251 | NH-NH | $CH_3$ | 3,5-($CH_3$)$_2$-Phenyl | 2-F, 6-Cl | 143-144°C |
| 252 | NH-NH | $CH_3$ | 2,6-$Cl_2$-Phenyl | 2-F, 6-Cl | 116-117°C |
| 253 | NH-NH | $CH_3$ | 2,5-$F_2$-Phenyl | 2-F, 6-Cl | 81-82°C |

## Patentansprüche

1. Verwendung von 3-arylsubstituierten 5-Alkyl-isoxazol-4-carbonsäurederivaten der allgemeinen Formel (I)

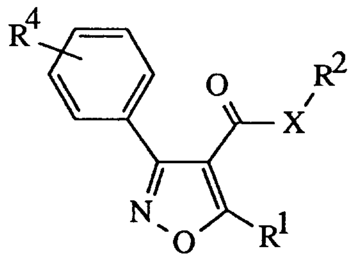

(I)

in welcher

$R^1$ für Alkyl oder Halogenalkyl steht,

$R^2$ für substituiertes $C_1$-$C_{20}$-Alkyl, $C_2$-$C_{18}$-Alkenyl, Cycloalkyl sowie für gegebenenfalls substituiertes Cycloalkylalkyl, Aralkyl, Heteroarylalkyl, Aryl, Heteroaryl steht, wobei als Substituenten genannt seien Halogen, Cyano, Nitro, Amino, Aminoacyl, Alkylamino, Alkyl, Halogenalkyl, Alalkyl, Hydroxy, Alkoxy, Halogenalkoxy, Aryloxy, Mercapto, Alkylthio, Halogenalkylthio, Alkylsulfinyl, Halogenalkylsulfinyl, Alkylsulfonyl, Halogenalkylsulfonyl, Aryl, Hetaryl, Arylthio oder Sulfonamidyl,

X für O, S, -NH-NH- oder für den Rest -N-$R^3$ steht,

$R^3$ für Wasserstoff, substituiertes $C_1$-$C_{20}$-Alkyl, $C_2$-$C_{18}$-Alkenyl, $C_1$-$C_6$-Halogenalkyl steht oder $R^2$ und $R^3$ gemeinsam mit dem angrenzenden N-Atom für einen 5-, 6 oder 7-gliedrigen Ring stehen, der gegebenenfalls auch durch O, S, N unterbrochen sein kann und gegebenenfalls durch $C_1$-$C_4$-Alkyl, Aralkyl oder Aryl substituiert ist,

$R^4$ für Wasserstoff, Halogen, Alkyl, Halogenalkyl steht,

zur Bekämpfung von Endoparasiten in der Medizin und Tiermedizin.

**2.** Neue 3-arylsubstituierte 5-Alkyl-isoxazol-4-carbonsäurederivate der allgemeinen Formel (I)

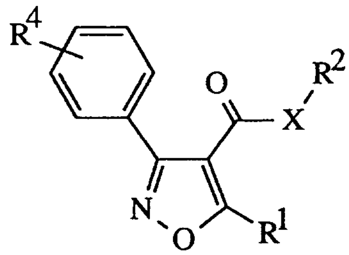

(I)

in welcher

$R^1$ für Alkyl oder Halogenalkyl steht,

$R^2$ für substituiertes $C_4$-$C_{20}$-Alkyl, $C_2$-$C_{18}$-Alkenyl, Cycloalkyl sowie für gegebenenfalls substituiertes Cycloalkylalkyl, Aralkyl, Heteroarylalkyl, Aryl, Heteroaryl steht, wobei als Substituenten genannt seien Cyano, Aminoacyl, Alkylamino, Alalkyl, Halogenalkoxy, Aryloxy, Mercapto, Alkylthio, Halogenalkylthio, Alkylsulfinyl, Halogenalkylsulfinyl, Alkylsulfonyl, Halogenalkylsulfonyl, Aryl, Hetaryl, Arylthio oder Sulfonamidyl,

X für O, S, -NH-NH- oder für den Rest -N-$R^3$ steht

$R^3$ für Wasserstoff, substituiertes $C_1$-$C_{20}$-Alkyl, $C_2$-$C_{18}$-Alkenyl, $C_1$-$C_6$-Halogenalkyl steht oder $R^2$ und $R^3$ gemeinsam mit dem angrenzenden N-Atom für einen 5-, 6 oder 7-gliedrigen Ring stehen, der gegebenenfalls auch durch O, S, N unterbrochen sein kann und gegebenenfalls durch $C_1$-$C_4$-Alkyl, Aralkyl oder Aryl substituiert ist,

$R^4$ für Wasserstoff, Halogen, Alkyl, Halogenalkyl steht.

**3.** Verfahren zur Herstellung der neuen 3-arylsubstituierten 5-Alkyl-isoxazol-4-carbonsäurederivate der Formel (I) gemäß Anspruch 2

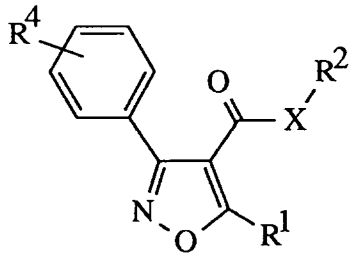

(I)

in welcher

X, $R^1$, $R^2$, $R^3$ und $R^4$ die unter Punkt 2 angegebene Bedeutung besitzen,

dadurch gekennzeichnet, daß man

a) Verbindungen der Formel (II)

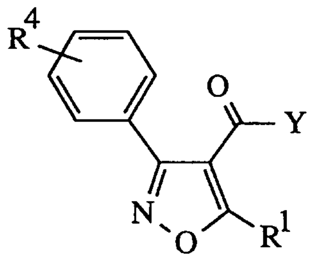

(II)

in welcher

$R^1$, $R^4$ die oben angegebene Bedeutung besitzen und

Y für eine geeignete Abgangsgruppe steht, mit Verbindungen der Formel (III)

$R^2$-X-H (III)

in welcher X, $R^2$ die oben angegebene Bedeutung besitzen,

umsetzt.

**4.** Endoparasitizide Mittel, gekennzeichnet durch einen Gehalt an mindestens einem 3-arylsubstituierten 5-Alkyl-isoxazol-4-carbonsäurederivat der Formel (I) gemäß Anspruch 1.

**5.** Verfahren zur Herstellung von endoparasitiziden Mitteln, dadurch gekennzeichnet, daß man 3-arylsubstituierte 5-Alkyl-isoxazol-4-carbonsäurederivate der Formel (I) gemäß Anspruch 1 mit Streckmitteln und/oder oberflächenaktiven Mitteln vermischt.

**6.** Verwendung von 3-arylsubstituierten 5-Alkyl-isoxazol-4-carbonsäurederivaten der Formel (I) gemäß Anspruch 1 zur Herstellung von endoparasitiziden Mitteln.

Europäisches Patentamt

# EUROPÄISCHER RECHERCHENBERICHT

Nummer der Anmeldung

EP 93 10 8786

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-A-3 247 454 (LABORATORIOS BAGO S.A.)<br>* Ansprüche 1,14 *<br>--- | 1,2 | C07D261/18<br>A61K31/42 |
| D,A | US-A-3 466 296 (DEAN C. PLEMMONS)<br>* das ganze Dokument *<br>--- | 1,2 | |
| A | EP-A-0 483 631 (BAYER AG)<br>* Ansprüche 1,5-8 *<br>--- | 1,4-6 | |
| A | SYNTHESIS.<br>Nr. 5, Mai 1984, STUTTGART DE<br>Seiten 413 - 417<br>JUAN CABRE ET AL 'New experimental strategies in amide synthesis using N,N-bis [2-oxo-3-oxazolidinyl]phosphorodiamidic chloride'<br>* das ganze Dokument *<br>--- | 2,3 | |
| D,A | JOURNAL OF THE CHEMICAL SOCIETY 1963, LETCHWORTH GB<br>Seiten 5838 - 5845<br>F.P.DOYLE ET AL 'Derivatives of 6-aminopenicillanic acid.Part VI.Penicillins from 3-and 5-phenylisoxazole-4-carboxylic acids and their alkyl and halogen derivatives'<br>*Seite 5838;Seiten 5842,5843*<br>--- | 1,2,3 | **RECHERCHIERTE SACHGEBIETE (Int. Cl.5)**<br>C07D<br>A61K |
| A | JOURNAL OF THE CHEMICAL SOCIETY 1965, LETCHWORTH GB<br>Seiten 5976 - 5983<br>J.C. HANSON ET AL 'Derivatives of 6-aminopenicillanic acid.Part VIII.Further analogues of 3-o-chlorophenyl-5-methyl-4-isoxazolylpenicillin'<br>*Seite 5976;Seiten 5979 und 5980,Table 1*<br>----- | 1-3 | |

Der vorliegende Recherchenbericht wurde für alle Patentansprüche erstellt

| Recherchenort | Abschlußdatum der Recherche | Prüfer |
|---|---|---|
| DEN HAAG | 13 AUGUST 1993 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)